# EUROPÄISCHE PATENTSCHRIFT

(11) **EP 0 467 175 B1**
(45) Veröffentlichungstag und Bekanntmachung des Hinweises auf die Patenterteilung: **20.09.1995**
(21) Anmeldenummer: 91111262.1
(22) Anmeldetag: 06.07.1991
(51) Int. Cl.: G01N 33/52, C12Q 1/37

(54) **Testkit zur Bestimmung eines Analyten in einer pastösen Probe, insbesondere in Stuhl**
Test kit for determining an analyte in a semi-solid sample, particularly in stools
Trousse pour la détermination d'une analyte dans un échantillon semi-solide, en particulier dans les selles

(30) Priorität: 17.07.1990 DE 4022655
(43) Veröffentlichungstag der Anmeldung: 22.01.1992
(73) Patentinhaber: BOEHRINGER MANNHEIM GMBH, 68298 Mannheim (DE)
(72) Erfinder: Steinbiss, Joachim, Dr., W-6143 Lorsch (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 271 854
- EP-A- 0 279 574
- EP-A- 0 291 843
- EP-A- 0 353 501
- EP-A- 0 353 570

## Beschreibung

Die Erfindung betrifft einen Testkit nach dem Oberbegriff des Anspruchs 1
Ein solcher Testkit ist aus der EP-A-0 291 843 bekannt, auf welche hier Bezug genommen wird. Der dort beschriebene Testkit dient ebenso wie die vorliegende Erfindung in erster Linie zur Analyse von Stuhl, ist jedoch auch für andere Probenmaterialien vorteilhaft einsetzbar, insbesondere Homogenisate tierischer und menschlicher Gewebeproben oder galenische Aufschlämmungen im Rahmen der pharmazeutischen Analytik. Allgemein bezieht sich die Erfindung auf die Analyse pastöser, streichfähiger Proben, welche feste Bestandteile enthalten, insbesondere für medizinische Zwecke. Im folgenden wird der Einfachheit halber, jedoch ohne Beschränkung der Allgemeinheit, nur auf die Analyse von Stuhl eingegangen.

Als Testkit werden Kombinationen von für eine Analyse notwendigen Reagenzien und Hilfsmitteln bezeichnet. Meist besteht ein Testkit aus mehreren Einheiten, es werden jedoch auch einstückige Analyseelemente für Stuhluntersuchungen angeboten, die ebenfalls als Testkit im Sinne der vorliegenden Erfindung anzusehen sind. Der in der genannten europäischen Patentanmeldung beschriebene Testkit ist insbesondere für immunologische Analysen von Stuhlbestandteilen (insbesondere von HSA, Humanserumalbumin) geeignet. Im Verhältnis zu den bis dahin bekannten immunologischen Stuhluntersuchungen zeichnet er sich vor allem durch eine einfache Handhabung aus. Die Stuhlprobe wird auf das Probenfeld aufgetragen, wobei sie teilweise in die poröse Probenschicht eindringt. Danach wird eine Eluationsflüssigkeit (beispielsweise wässrige Pufferlösung) in dem Eluationsmittelauftragsbereich auf die Flüssigkeitstransportstrecke, welche vorzugsweise aus einem einzigen Schichtmaterial besteht, aufgebracht. Sie chromatografiert die Flüssigkeitsstrecke entlang, wobei sie aus der Probe lösliche Bestandteile eluiert. Das Eluat chromatografiert aufgrund der in der Flüssigkeitstransportstrecke herrschenden Kapillarkräfte weiter in den Eluataufnahmebereich. Dort sind Analysemittel vorgesehen, die mit dem Analyten reagieren und ein Nachweissignal erzeugen, welches vorzugsweise in einer Farbänderung besteht. Es sind aber auch andere Nachweissignale, (z.B. Fluoreszenz) je nach den verwendeten Analysemitteln möglich.

Der vorliegenden Erfindung liegt die Aufgabe zugrunde, einen Testkit für die Analyse pastöser Proben zur Verfügung zu stellen, welcher verbesserte Analyseeigenschaften hat.

Die Aufgabe wird bei einem Testkit der eingangs bezeichneten Art durch die kennzeichnenden Merkmale des Anspruchs 1 gelöst.

Durch die Verzögerungsstrecke wird die Flüssigkeitstransportgeschwindigkeit in der Probenschicht gezielt reduziert. Betrachtet man die Probenschicht als separates Flüssigkeitstransportelement, so wird die Strömungsgeschwindigkeit primär durch die Flüssigkeitstransporteigenschaften ihres Materials bestimmt. Diese sind einerseits abhängig von den Kapillarkräften in der Schicht, die durch die Oberflächeneigenschaften des Schichtmaterials und die Größe der Kapillarspalte bestimmt werden und andererseits sind sie bestimmt durch den Strömungswiderstand in der Schicht. Diese Parameter können in der Praxis jedoch nicht unabhängig voneinander festgelegt und optimiert werden. Insbesondere ist das Schichtmaterial auch nach anderen Einflußgrößen auszuwählen, wie beispielsweise Festigkeit, relativ geringe Schichtstärke (vorzugsweise weniger als 0,5 mm, besonders bevorzugt weniger als 0,3 mm) und ausreichende Porosität für das Eindringen der Probe.

Bei dem vorbekannten Testkit schließt sich das Probenfeld (beziehungsweise in einer bevorzugten Ausführungsform das erste von mehreren Probenfeldern) unmittelbar an den Eluationsmittelauftragsbereich an. Dadurch ist die Strömungsgeschwindigkeit, mit der das Eluationsmittel in der Probenschicht durch die aufgetragene Probe strömt, praktisch nur von deren eigenen Eigenschaften bestimmt. Bei der vorliegenden Erfindung ist die Flüssigkeitstransportgeschwindigkeit der Verzögerungsstrecke erheblich niedriger. Da die gesamte Flüssigkeitstransportstrecke des Testkits in guter Näherung einen geschlossenen Strömungspfad bildet, ist die Strömungsgeschwindigkeit (ausgedrückt als Massentransport, beispielsweise in µl/sec) in ihr überall gleich. Die Verzögerungsstrecke erlaubt also, die Flüssigkeitstransportgeschwindigkeit innerhalb der Probenschicht im Bereich des Probenfeldes unabhängig von deren eigenen Flüssigkeitstransporteigenschaften auf eine für den jeweiligen Test optimalen Wert festzulegen.

Eine Verzögerungsstrecke im Sinne der vorliegenden Erfindung hat die Eigenschaft, daß sie, isoliert betrachtet, eine geringere Flüssigkeitstransportgeschwindigkeit für das Eluationsmittel aufweist, als die Probenschicht im Bereich des Probenfeldes (wiederum isoliert betrachtet). Vorzugsweise sollte die Strömungsgeschwindigkeit in der Verzögerungsstrecke mindestens um einen Faktor 2, vorzugsweise mindestens um einen Faktor 5 kleiner sein, als die Strömungsgeschwindigkeit im Bereich eines Probenfeldes der isoliert untersuchten Probenschicht.

Die reduzierte Flüssigkeitstransportgeschwindigkeit in der Verzögerungsstrecke kann auf verschiedenerlei Weise realisiert werden. Im einfachsten Fall kann die Verzögerungsstrecke einfach aus einem verlängerten Streckenabschnitt zwischen Eluationsmittelauftragsbereich und Probenfeld (bzw. bei mehreren Probenfeldern dem ersten Probenfeld) bestehen. Bevorzugt ist sie länger als die Längsdimension des Probenfeldes in Strömungsrichtung, besonders bevorzugt mindestens doppelt so lang.

Die Verzögerungsstrecke kann auch aus einem von der Probenschicht verschiedenen kapillaraktiven Schichtmaterial bestehen, welches die Flüssigkeit langsamer transportiert. Dies ist jedoch herstellungstechnisch aufwendig, weil in dem Testkit entlang der Flüssigkeitstransportstrecke mehrere Schichtmaterialien positioniert und so miteinander verbunden werden müssen, daß die Flüssigkeit von der einen Schicht in die andere übertreten kann ("Fluidkontakt").

Besonders bevorzugt ist deshalb die Verwendung eines einheitlichen Schichtmaterials für Verzögerungsstrecke und Probenschicht, wobei im Bereich der Verzögerungsstrecke eine Verengung vorgesehen ist. Die Verengung kann sowohl aus einer oder mehreren Eindrückungen senkrecht zur Flächenausdehnung des Schichtmaterials als auch aus einem oder mehreren Abschnitten von reduzierter Breite des Schichtmaterials bestehen.

Die verschiedenen Realisierungen der Verzögerungsstrecke können auch miteinander kombiniert werden.

Durch die Erfindung wird eine verbesserte Analysequalität erreicht. Die reduzierte Strömungsgeschwindigkeit des Eluationsmittel in der Probenschicht führt zu einer Erhöhung der Analytkonzentration. Im Rahmen der vorliegenden Erfindung wurde festgestellt, daß dies für die Analysequalität sehr vorteilhaft ist, obwohl einerseits hochempfindliche Reagenzsysteme zur Verfügung stehen, um die in Frage kommenden Analyten mit außerordentlich hoher Empfindlichkeit nachzuweisen, und obwohl andererseits die Verzögerungsstrecke den Zeitbedarf für die Analyse erhöht und eine kompliziertere Gestaltung der Flüssigkeitstransportstrecke erfordert. Dadurch, daß erfindungsgemäß die Eluationseigenschaften verbessert werden und zugleich ein verhältnismäßig unempfindliches Nachweissystem eingesetzt wird, wird die Zahl falsch positiver Befunde wesentlich reduziert und dennoch die erforderliche Nachweissicherheit gewährleistet, also falsch negative Befunde weitgehend eliminiert.

Außerdem führt die Erfindung zu einer verbesserten Abtrennung der für die Analyse störenden gefärbten Bestandteile des Stuhls.

Ein besonderer Vorzug der Erfindung besteht darin, daß das Analyseergebnis weitgehend unabhängig vom Trocknungszustand der Probe ist. Während bei den vorbekannten Analyseelementen mit einer verhältnismäßig frischen Probe die Wahrscheinlichkeit falsch positiver Befunde anstieg, bzw. (bei reduzierter Nachweisempfindlichkeit des Analysesystems) die Wahrscheinlichkeit falsch negativer Befunde bei stark eingetrockneten Proben wuchs, werden erfindungsgemäß bei weitgehend frischen und bei längere Zeit gelagerten Proben nahezu die gleichen Ergebnisse erzielt.

Erfindungsgemäß können auch relativ unempfindliche, nicht-immunologische, insbesondere enzymatische Analysen mit hoher Zuverlässigkeit durchgeführt werden.

Überraschenderweise zeigt sich, daß die Reduzierung der Strömungsgeschwindigkeit zwar zu einer Anreicherung des Analyten, jedoch nicht zur einer praktisch störenden Konzentrationserhöhung der die Analyse störenden Stuhlbestandteile führt.

Die Erfindung wird im folgenden anhand eines in den Figuren schematisch dargestellten Ausführungsbeispiels näher erläutert; es zeigen:
- Fig. 1: Eine Prinzipdarstellung eines erfindungsgemäßen Testkits im Schnitt,
- Fig. 2: die Probensammeleinheit einer bevorzugten Ausführungsform in perspektivischer Darstellung von ihrer Patientenseite,
- Fig. 3: eine Probensammeleinheit gemäß Fig. 2 in perspektivischer Darstellung von der Arztseite her einschließlich zu dem Testkit gehörigen Testträgern,
- Fig. 4: eine Aufsicht auf einen Kartonzuschnitt zur Herstellung einer Probensammeleinheit nach den Figuren 2 und 3,
- Fig. 5: ein Faltschema zu Fig. 4,
- Fig. 6: einen Längsschnitt durch eine Probensammeleinheit nach den Figuren 2 bis 5.

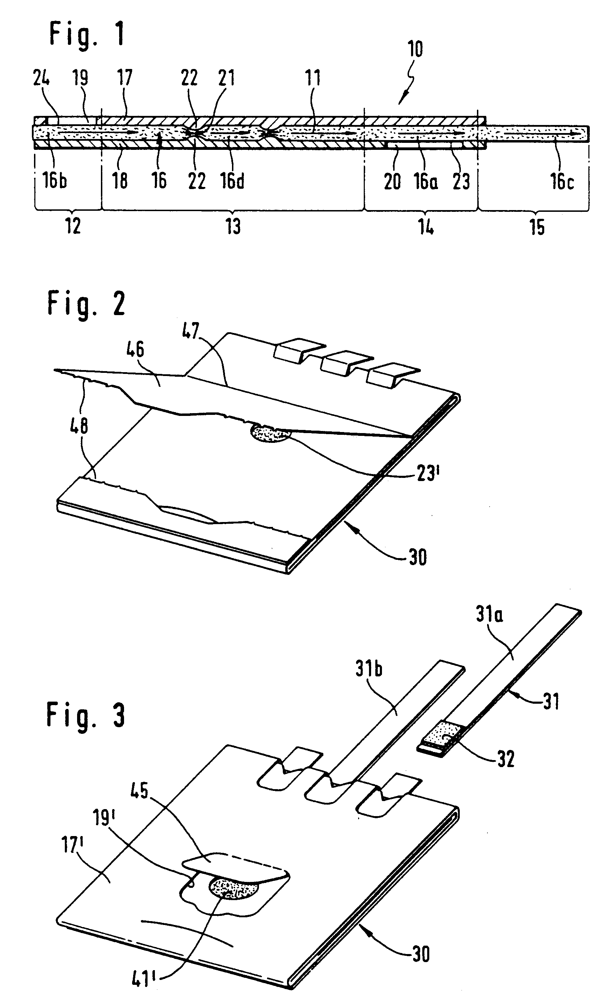

Das in Figur 1 dargestellte Labormuster eines Testkit 10 weist eine durch den Pfeil 11 symbolisierte Flüssigkeitstransportstrecke auf, welche von einem Eluationsmittelauftragsbereich 12 über eine Verzögerungsstrecke 13 und einen Probenauftragsbereich 14 zu einem Eluataufnahmebereich 15 führt.

Die Flüssigkeitstransportstrecke 11 wird im dargestellten bevorzugten Fall von einem einzigen durchgehenden Einsatzteil 16 aus einem porösen, kapillaren Schichtmaterial gebildet, welches zwischen zwei Abdeckteilen 17 und 18 sandwichartig eingeschlossen ist. Diese Ausführungsform ist besonders einfach herzustellen. Im Rahmen der Erfindung können jedoch auch unterschiedliche Schichtmaterialien in den Bereichen 12 bis 16 Anwendung finden, die in Fluidkontakt zueinander stehen. Der Fluidkontakt kann dabei nicht nur durch kapillaraktive poröse Schichtmaterialien, sondern auch durch Kapillarspalten oder Röhren hergestellt sein. Die einzelnen Abschnitte des Schichtmaterials in den Bereichen 12 bis 15 werden als Ansaugschicht 16b, Verzögerungsschicht 16d, Probenschicht 16a und Eluataufnahmeschicht 16c bezeichnet.

Das erste Abdeckteil 17 und das zweite Abdeckteil 18 weisen jeweils eine Ausnehmung 19 bzw. 20 auf, durch die die Flüssigkeitstransportstrecke im Eluationsmittelaufgabebereich 12 (Ansaugschicht 16b) bzw. im Probenauftragsbereich 14 (Probenschicht 16a) zugänglich ist. Die Abdeckteile 17,18 sind aus einem feuchtigkeitsbeständigen Schichtmaterial, beispielsweise beschichtetem Karton oder einer stabilen Kunststoffolie hergestellt. Sie können wahlweise auch als Profilteile ausgebildet sein (beispielsweise wie in der EP-A-0 291 843 dargestellt).

Die Verzögerungsschicht 16d besteht in dem in Figur 1 dargestellten bevorzugten Fall aus dem gleichen kapillaraktiven flachen Schichtmaterial (des Einsatzteils 16) wie die übrige Transportstrecke. Sein Flüssigkeitstransportquerschnitt ist durch Eindrückungen 21, an denen das Schichtmaterial senkrecht zu seiner Flächendimension zusammengedrückt ist, verengt. Zusätzlich basiert die Verzögerungswirkung der Verzögerungsstrecke 13 darauf, daß sie relativ lang ist. Vorzugsweise beträgt ihre Länge mindestens 25% der gesamten Flüssigkeitstransportstrecke von dem Eluationsmittelauftragsbereich 12 bis zu dem Eluataufnahmebereich 15.

Bei der dargestellten Ausführungsform werden die Eindrückungen 21 durch entsprechende erhabene Profilierungen 22 an den Abdeckteilen 17, 18 fixiert. Dies ist jedoch nur erforderlich, wenn das Material, aus dem die Verzögerungsschicht besteht, so elastisch ist, daß die Eindrückungen 21 ohne eine entsprechende Fixierung nicht erhalten bleiben.

Zur Durchführung einer Analyse wird der in Figur 1 dargestellte Testkit mit dem zweiten Abdeckteil 18 nach oben (also umgekehrt wie dargestellt) gehalten. Die Probe wird auf das von der Ausnehmung 20 umrahmte Probenfeld aufgetragen, wobei sie in den darunter befindlichen Abschnitt der Flüssigkeitstransportstrecke 11 (Probenschicht 16a) teilweise eindringt.

Zur Auswertung wird der Testkit mit dem ersten Abdeckteil 17 nach oben gehalten, so daß ein Eluationsmittel (vorzugsweise eine wässrige Lösung mit Hilfsreagenzien, beispielsweise Netzmittel und Puffer) durch die Ausnehmung 19 auf die darunter befindliche Ansaugschicht 16b aufgebracht werden kann. Das Eluationsmittel, welches in ausreichender Menge aufgetragen wird, um die gesamte kapillaraktive Flüssigkeitstransportstrecke 11 zu füllen, wird durch Kapillarkräfte entlang der Verzögerungsstrecke 13 zu dem Probenauftragsbereich 14 (Probenschicht 16a) transportiert. Dabei wird die Strömungsgeschwindigkeit derartig verlangsamt, daß sie im Bereich der Probenschicht 16a erheblich vermindert ist. Dadurch wird eine wesentliche verbesserte Eluation in dem Probenauftragsbereich 14 erreicht.

Bei der in Figur 1 dargestellten Ausführungsform erfolgt der Nachweis des Analyten mit Hilfe der Eluataufnahmeschicht 16c. Sie kann abgetrennt und mit Hilfe eines zu dem Testkit gehörigen Reagenziensatzes analysiert werden. Alternativ ist die Eluataufnahmeschicht 16c selbst mit den notwendigen Reagenzien zur Erzeugung eines für die Analyse spezifischen Nachweissignals ausgerüstet, welches in bekannter Weise (im Falle eines Farbumschlages üblicherweise reflexionsphotometrisch) ausgewertet wird.

Die Figuren 2 bis 6 zeigen eine bevorzugte Ausführungsform, die sich sowohl durch ihre Funktion (genaue und reproduzierbare Analyseresultate) als auch durch eine einfache und damit kostengünstige Herstellbarkeit auszeichnet.

Der Testkit umfaßt eine Probensammeleinheit 30 und spezifisch darauf abgestimmte Testträger 31 (Fig. 3), welche zweckmäßigerweise ähnlich einem konventionellen Teststreifen ausgebildet sind. Sie enthalten in einer oder mehreren übereinander oder nebeneinander angeordneten Testschichten ein Reagenzsystem zur Bestimmung eines bestimmten Analyten. In der Figur ist nur eine Testschicht 32 angedeutet.

Innerhalb eines erfindungsgemäßen Testkits werden bevorzugt mehrere Typen von Testträgern 31a, 31b verwendet, die zur Bestimmung unterschiedlicher Analyten (Parameter) dienen und damit auf sehr einfache Weise eine Profilanalyse des Stuhls ermöglichen. Dadurch läßt sich eine wesentlich exaktere diagnostische Aussage über das Vorliegen einer bestimmten Erkrankung machen. Besonders vorteilhaft ist, daß das Profil selektiv ist, d.h. daß vom Arzt jeweils nur die Parameter bestimmt werden müssen, die im Zusammenhang mit der vermuteten Diagnose erforderlich sind. Außerdem ist die Analysedurchführung praktisch ebenso einfach wie bei den bisher üblichen Stuhlbluttests, die im Rahmen der Gesundheitsvorsorge in sehr großen Zahlen durchgeführt werden.

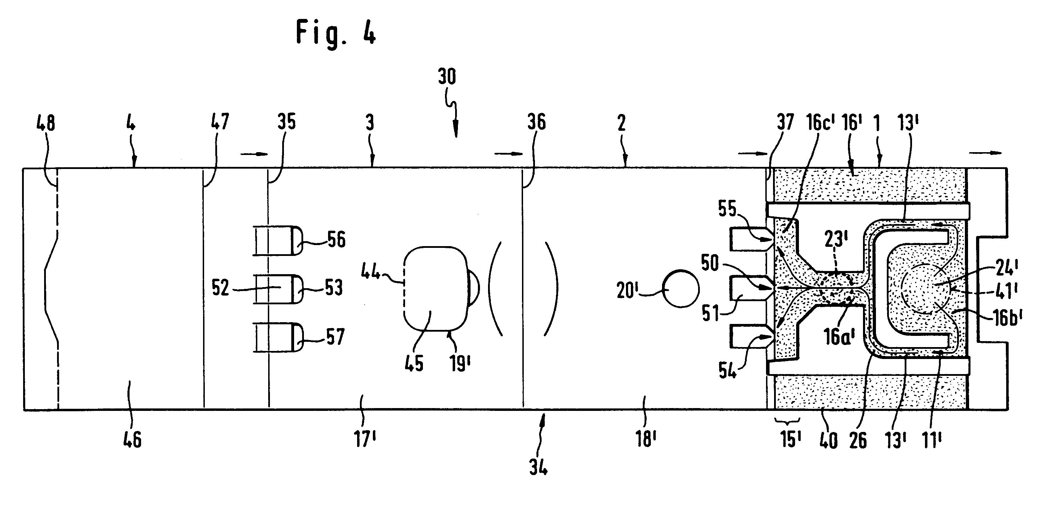

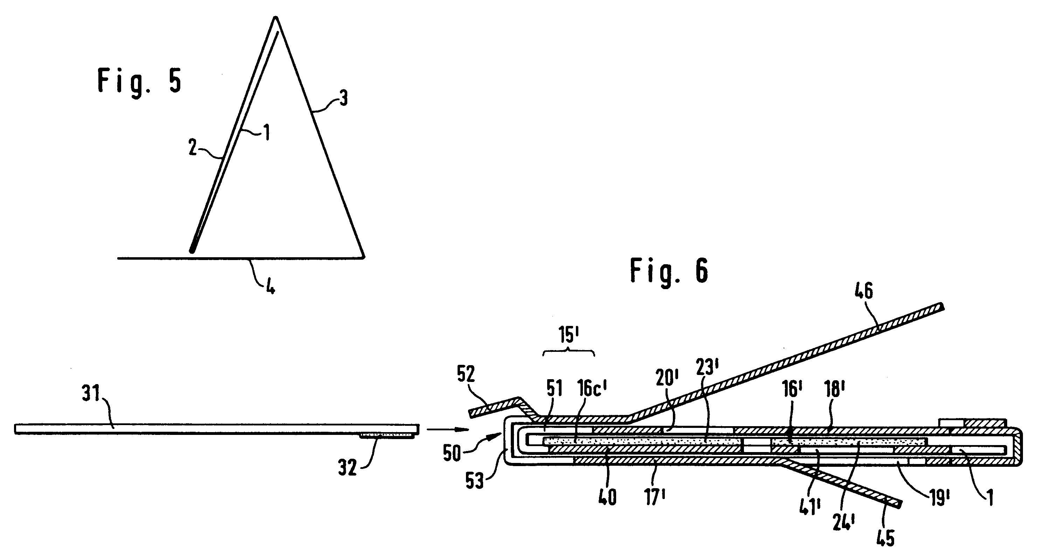

Die Probensammeleinheit 30 besteht nur aus zwei Teilen, nämlich einem als Faltteil 34 bezeichneten Zuschnitt aus wasserfest beschichtetem, dünnem (maximal ca. 500 g/m²) Karton und einem Einsatzteil 16' aus einem porösen, kapillaren Schichtmaterial (Fig. 4 und Fig. 6). Als kapillares Schichtmaterial eignen sich grundsätzlich sowohl Naturstoffe als auch (bevorzugt) hydrophile Kunststoffe, die zur Erzeugung der kapillarformenden Strukturen als Papiere, Vliese, Gewebe oder entsprechend poröse und strukturierte Folien verarbeitet sind. Ein Vlies aus hydrophilen Kunststoffasern ist besonders bevorzugt.

Das Faltteil 34 weist drei Hauptfaltlinien 35, 36 und 37 auf, durch die vier Abschnitte 1 bis 4 abgeteilt sind, welche bei der Herstellung der Probensammeleinheit 30 in der in Fig. 5 dargestellten Weise gefaltet werden.

Der Abschnitt 1 des Faltteils 34 dient hauptsächlich als Tragteil 40 für das Einsatzteil 16'. Das Einsatzteil 16' ist an dem Tragteil 40 derartig befestigt, daß seine kapillaraktiven Eigenschaften durch die Befestigung nicht beeinträchtigt werden. Geeignet ist beispielsweise eine punkt- oder streifenweise Verklebung am Rand des Einsatzteils 16'.

Die Abschnitte 2 und 3 bilden das zweite Abdeckteil 18' mit der Ausnehmung 20', die das Probenfeld 23' umrahmt, bzw. das erste Abdeckteil 17' mit der Ausnehmung 19', die das Eluatmittelauftragsfeld 24' zugänglich macht.

Dieser Schichtaufbau ist in Fig. 6 am deutlichsten zu erkennen, wobei die Schichtstärken übertrieben dick dargestellt sind. die tatsächliche Dicke des Schichtmaterials, aus dem das Faltteil 34 hergestellt ist, liegt vorzugsweise unter 0,5 mm, so daß sich ein Briefchen mit einer Stärke von weniger als etwa 2 mm daraus falten läßt.

Eine bevorzugte Gestaltung der Flüssigkeitstransportstrecke 11', insbesondere der Verzögerungsstrecke 13', ist in Fig. 4 zu erkennen. Das Eluatauftragsfeld 24' in der Ansaugschicht 16b' ist verhältnismäßig ausgedehnt. Von hier führt der Flüssigkeitstransportweg 11' über eine zweigeteilte Teilstrecke 26 zu der Probenschicht 16a' mit dem (in Fig. 4 durch Punkte angedeuteten) Probenfeld 23' und von dort zu einer Eluataufnahmeschicht 16c'. Charakteristisch für die dargestellte bevorzugte Ausführungsform ist, daß die Breite der Teilstrecke 26 (Summe beider Zweige) kleiner als die Breite des Flüssigkeitstransportquerschnittes im Bereich des Probenfeldes 23' ist. Außerdem ist sie sehr lang (ca. 50% der gesamten Flüssigkeitstransportstrecke). Um dennoch eine handliche Gesamtgröße der Probensammeleinheit 30 zu ermöglichen, weist die Flüssigkeitstransportstrecke 11' Windungen auf, bei denen die Flüssigkeitstransportrichtung sich mehrfach ändert.

Das Eluationsmittelauftragsfeld 24' ist durch die Ausnehmung 19' in dem ersten Abdeckteil 17' und eine weitere Ausnehmung 41' in dem Tragteil 40' von der Arztseite (Fig. 3) der Probensammeleinheit her zugänglich. Die Ausnehmung 19' ist mit einer um eine Knicklinie 44 schwenkbaren Klappe 45 verschließbar.

Das Probenfeld 23' ist über die Ausnehmung 20' in dem zweiten Abdeckteil 18' von der Patientenseite (Fig.2) her zugänglich. Es ist mit einer Klappe 46 verschließbar, die um eine Knicklinie 47 schwenkbar ist. Sie ist im ungebrauchten Zustand der Probensammeleinheit 30 an ihrer Vorderkante über eine Abreißlinie 48 geschlossen.

In dem Eluataufnahmebereich 15' sind insgesamt mit 50 bezeichnete Eluattransfermittel vorgesehen. Die Eluattransfermittel 50 und die Testträger 31 sind so aufeinander abgestimmt, daß in dem Eluataufnahmebereich 15' vorhandenes Eluat auf die mindestens eine Testschicht 32 des Testträgers transferiert werden kann.

Die Eluattransfermittel 50 umfassen im dargestellten Fall die Eluataufnahmeschicht 16c', eine Distanzausnehmung 51 in der zweiten Abdeckschicht 18' und eine im Ausgangszustand der Probensammeleinheit 30 mit einer Klappe 52 verschlossene Testträgeröffnung 53. Wie aus Fig. 6 zu erkennen ist, sind die Dimensionen so aufeinander abgestimmt, daß ein durch die Öffnung 53 in die Distanzausnehmung 51 eingeschlossener Testträger mit seinem Testfeld 32 in Fluidkontakt zu der Eluataufnahmeschicht 16c' steht.

Im dargestellten bevorzugten Fall ist die Eluataufnahmeschicht 16c' derartig verbreitet, daß mit Hilfe von drei Eluattransfereinrichtungen 50, 54 und 55 ein Fluidkontakt zu drei in die Öffnungen 53, 56 und 57 eingesteckten Testträgern hergestellt werden kann, welche sich hinsichtlich des Analyten, für die sie spezifisch sind, unterscheiden. Hierdurch ist die Bestimmung eines Analyseprofils aus mehreren Parametern möglich. Eine besonders bevorzugte Kombination umfaßt folgende Bestimmungen:
1. Konventioneller Nachweis des okkulten Blutes, insbesondere mit Hilfe eines Hämoglobin-Peroxidasetests.
2. Immunologische Bestimmung von HSA gemäß EP-A-291 843.
3. Nachweis von Leukozyten über einen enzymatischen Test der Leukozyten-Elastase.

Die Bestimmung von Leukozyten durch Nachweis ihrer Elastase-Aktivität ist für die Urinanalyse entwickelt worden. Dabei wurde auch bereits die Möglichkeit angedeutet, Leukozyten im Stuhl nachzuweisen (EP-A-00 12 957). Obwohl die diagnostische Relevanz dieses Nachweises, welcher insbesondere wertvolle Hinweise auf das Vorliegen entzündlicher Erkrankungen geben kann, unbestritten ist, hat es bisher an einer einfach handhabbaren und zuverlässigen Methodik gefehlt, diesen Nachweis auch tatsächlich zu ermöglichen. Ein Testkit für eine derartige Bestimmung wird durch die vorliegende Erfindung zur Verfügung gestellt. Dabei hat sich überraschenderweise ergeben, daß besonders gute Ergebnisse hinsichtlich Empfindlichkeit einerseits und Selektivität andererseits (Vermeidung von falsch negativen und falsch positiven Befunden) erreicht wird, wenn man auf Basis der vorliegenden Erfindung für eine verhältnismäßig hohe Konzentration des Analyten in dem Eluat sorgt und andererseits mit einem verhältnismäßig unempfindlichen Leukozytennachweis arbeitet. Als besonders geeignetes Nachweisverfahren hat sich dabei das in der EP-B-00 12 957 beschriebene Verfahren einschließlich der in der EP-B-00 14 929 beschriebenen Verbesserungen erwiesen. Vorzugsweise wird die Nachweisgrenze der Leukozyten auf mehr als 500 Leukozyten/µl, besonders bevorzugt mehr als tausend Leukozyten/µl eingestellt.

Die Durchführung der Analyse ist sehr einfach:
Der Patient trägt die Stuhlprobe mit einem Spatel durch die Ausnehmung 20' auf die Probenfläche 23' auf und schließt die Klappe 46. Die Probensammeleinheit 30 ist dabei so gestaltet, daß in geringem Umfang Luft an die Probe gelangt, so daß diese bei Lagerung eintrocknet.

Zur Untersuchung der Probe werden die Testträgeröffnungen 53, 56 und 57 und die das Eluationsmittelauftragsfeld 24' verschließende Klappe 45 geöffnet. Je nach dem gewünschten Analyseprofil werden ein oder mehrere Testträger in die Eluattransfereinrichtungen 50, 54 und 55 eingeführt. Eine abgemessene Menge Eluationsmittel wird auf das Eluationsmittelauftragsfeld 24' aufdosiert. Der Testträger 31 wird zu einem vorbestimmten Zeitpunkt entnommen und das Nachweissignal wird visuell oder mit einem Gerät ausgewertet.

## Patentansprüche

1. Testkit zur Bestimmung eines Analyten in einer pastösen Probe, insbesondere in Stuhl, umfassend
eine kapillaraktive Flüssigkeitstransportstrecke für ein Eluationsmittel (11), die von einem Eluationsmittelauftragsbereich (12) durch eine in ihrer Längsrichtung durchströmte Probenschicht (16a) zu einem Eluataufnahmebereich (15) fuhrt, wobei die Probenschicht (16a) ein Probenfeld (23) zum Auftragen der Probe aufweist und so porös ist, daß die Probe beim Auftragen auf das Probenfeld teilweise in sie eindringt und
Analysemittel (16c, 31) mit Reagenzien, die mit dem Analyten reagieren und eine ein Nachweissignal erzeugende Komponente einschließen,
**dadurch gekennzeichnet, daß**
zur gezielten Reduzierung der Flüssigkeitstransportgeschwindigkeit in der Probenschicht zwischen dem Eluationsmittelzufuhrbereich (12) und dem Probenfeld (23) eine Verzögerungsstrecke (13) vorgesehen ist, die eine geringere Flüssigkeitstransportgeschwindigkeit für das Eluationsmittel aufweist als die Probenschicht im Bereich des Probenfeldes, wenn man jeweils isoliert die Flüssigkeitstransportgeschwindigkeit der Probenschicht und der Verzögerungsstrecke als Massentransport des Eluationsmittels mißt.

2. Testkit nach Anspruch 1, **dadurch gekennzeichnet**, daß die Verzögerungsstrecke (13) erheblich länger als die Längsdimension des Probenfeldes (23) in Strömungsrichtung ist.

3. Testkit nach Anspruch 1 oder 2, **dadurch gekennzeichnet**, daß die Verzögerungsstrecke (13) aus einem kapillaraktiven flachen Schichtmaterial (16a) besteht und eine Verengung des Flüssigkeitstransportquerschnitts aufweist.

4. Testkit nach Anspruch 3, **dadurch gekennzeichnet**, daß die Verengung eine Eindrückung (21) ist, an der das Schichtmaterial senkrecht zu seiner Flächendimension zusammengedrückt ist.

5. Testkit nach Anspruch 3 oder 4, **dadurch gekennzeichnet**, daß die Verengung eine Teilstrecke (26) mit in Relation zu der Probenschicht (16a) reduzierter Breite ist.

6. Testkit nach einem der vorhergehenden Ansprüche, **dadurch gekennzeichnet**, daß die Flüssigkeitstransportstrecke (11) Teil einer Probensammeleinheit (30) ist, in dem Eluataufnahmebereich (15) eine Eluattransfereinrichtung (50) vorgesehen ist, die Analysemittel einen Testträger (31) mit mindestens einer Testschicht (32) einschließen und der Testträger (31) und die Probensammeleinheit (30) so aufeinander abgestimmt sind, daß in dem Eluataufnahmebereich (15) vorhandenes Eluat mittels der Eluattransfereinrichtung (50) auf die mindestens eine Testschicht (32) des Testträgers (31) transferiert wird.

7. Testkit nach Anspruch 6, **dadurch gekennzeichnet**, daß der Eluataufnahmebereich (15) eine Mehrzahl von Eluattransfereinrichtungen (50,54,55) aufweist, die jeweils mit der Flüssigkeitstransportstrecke (11) in Fluidkontakt stehen.

8. Testkit nach einem der vorhergehenden Ansprüche, **dadurch gekennzeichnet**, daß die Flüssigkeitstransportstrecke (11) an einem Einsatzteil (16) aus einem porösen kapillaren Schichtmaterial ausgebildet ist, das Einsatzteil 16 zwischen einem ersten Abdeckteil (17) und einem zweiten Abdeckteil (18) sandwichartig eingeschlossen ist und die Abdeckteile (17,18) in dem Eluationsmittelaufgabebereich (12) und dem Probenauftragsbereich (14) jeweils mindestens eine Ausnehmung (19,20) haben, durch die ein Teil der an dem Einsatzteil (16) ausgebildeten Flüssigkeitstransportstrecke (11) zugänglich ist.

9. Testkit nach Anspruch 8, **dadurch gekennzeichnet**, daß das Einsatzteil (16') aus porösem Schichtmaterial auf einem Tragteil (40) aus wasserfestem Schichtmaterial befestigt ist, welches zusammen mit dem Einsatzteil (16') zwischen den Abdeckteilen (17',18') eingeschlossen ist.

10. Testkit nach einem der vorhergehenden Ansprüche, **dadurch gekennzeichnet**, daß die Analysemittel ein Reagenzsystem zur Bestimmung einer Elastase einschließen.

## Claims

1. Test kit for determining an analyte in a pasty sample, in particular in stool, comprising a capillary-active fluid transport path for an eluant (11), which path leads from an eluant feed zone (12) via a sample layer (16a), penetrating the layer in its longitudinal direction, to an eluate reception zone (15), wherein the sample layer (16a) is provided with a sample field (23) for the application of the sample and is so porous that the sample applied to the sample field partially penetrates into it, and analysis means (16c, 31) containing reagents which react with the analyte and comprise a component producing a test signal,
**characterized in that**
a delay section (13) is provided in the sample layer between the eluant feed zone (12) and the sample field (23) for reducing the fluid transport rate in a controlled manner, the fluid transport rate of the delay section for the eluant being lower than that of the sample layer in the region of the sample field, the flow rates in the sample layer and in the delay section being in each case measured in isolation as mass transfer of the eluant.

2. Test kit according to claim 1, **characterized in that** the delay section (13) is substantially longer than the longitudinal dimension of the sample field (23) in the flow direction.

3. Test kit according to claim 1 or 2, **characterized in that** the delay section (13) consists of a capillary-active, flat layer material (16a) and comprises a narrowing of the fluid transport cross-section.

4. Test kit according to claim 3, **characterized in that** the narrowing is a constriction (21) at which the layer material is compressed perpendicular to its surface dimension.

5. Test kit according to claim 3 or 4, **characterized in that** the narrowing is a sub-section (26) of reduced width in relation to the sample layer (16a).

6. Test kit according to any one of the preceding claims, **characterized in that** the fluid transport path (11) is part of a sample collecting unit (30), an eluate transfer device (50) is provided in the eluate reception zone (15), the analysis means include a test carrier (31) with at least one test layer (32), and the test carrier (31) and the sample collecting unit (30) are adapted to one another so that eluate present in the eluate reception zone (15) is transferred by means of the eluate transfer device (50) to the at least one test layer (32) of the test carrier (31).

7. Test kit according to claim 6, **characterized in that** the eluate reception zone (15) comprises a plurality of eluate transfer devices (50, 54, 55) each of which is in fluid communication with the fluid transport path (11).

8. Test kit according to any one of the preceding claims, **characterized in that** the fluid transport path (11) is formed on an insert part (16) consisting of a porous, capillary layer material, the insert part 16 is encased like a sandwich between a first cover part (17) and a second cover part (18), and the cover parts (17, 18) have in the eluant feed zone (12) and the sample application zone (14) in each case at least one recess (19, 20) through which part of the fluid transport path (11) formed on the insert part (16) is accessible.

9. Test kit according to claim 8, **characterized in that** the insert part (16') of porous layer material is fixed to a bearing part (40) of water-proof layer material, which is encased between the cover parts (17', 18') together with the insert part (16').

10. Test kit according to any one of the preceding claims, **characterized in that** the analysis means include a reagent system for determining an elastase.

## Revendications

1. Trousse pour la détermination d'une analyte dans un échantillon semi-solide, en particulier dans les selles, comprenant une conduite de transport de liquide tensio-actif pour un agent d'éluation (11), conduite qui mène d'une zone d'application de l'agent d'éluation (12), à travers une couche d'échantillon (16a) traversée dans son sens longitudinal, à une zone de réception du produit élué (15), la couche d'échantillon (16a) présentant un champ d'essai (23) pour appliquer l'échantillon et étant si poreuse que l'échantillon y pénètre partiellement lors de son application sur le champ d'essai, et des agents d'analyse (16c, 31) avec des réactifs qui réagissent à l'analyte et incluent un composant produisant un signal d'identification, caractérisée en ce que, pour la réduction recherchée de la vitesse de transport du liquide dans la couche d'échantillon, entre la zone d'amenée de l'agent d'éluation (12) et le champ d'essai (23) est prévu un parcours de retard (13) qui présente, pour l'agent d'éluation, une vitesse de transport plus faible que la couche d'essai dans la zone du champ d'essai lorsque l'on mesure a chaque fois séparément la vitesse de transport du liquide de la couche d'échantillon et du parcours de retard en tant que transport en masses de l'agent d'éluation.

2. Trousse selon la revendication 1, caractérisée en ce que le parcours de retard (13) est considérablement plus long que la dimension longitudinale du champ d'essai (23) dans le sens d'écoulement.

3. Trousse selon les revendications 1 ou 2, caractérisée en ce que le parcours de retard (13) se compose d'une couche plate tensio-active (16a) et présente une contraction de la section droite du transport du liquide.

4. Trousse selon la revendication 3, caractérisée en ce la contraction est une empreinte (21) contre laquelle le matériau stratifié est tassé perpendiculairement à à sa dimension superficielle.

5. Trousse selon les revendications 3 ou 4, caractérisée en ce que la contraction est un segment partiel (26) de largeur réduite en relation avec la couche d'échantillon (16a).

6. Trousse selon l'une des revendications précédentes, caractérisée en ce que le parcours de transport de liquide (11) correspond à une partie d'une unité collectrice d'échantillons (30), en ce qu'un dispositif de transfert du produit élué (50) est prévu dans la zone de réception du produit élué (15), en ce que les agents d'analyse incluent un porteur de test (31) avec au moins une couche d'essai (32) et en ce que le support de test (31) et l'unité collectrice d'échantillons (30) sont adaptés de façon telle l'un à l'autre que le produit élué présent dans la zone de réception du produit élué (15) est transféré sur au moins l'une des couches d'essai (32) du porteur de test (31), au moyen du dispositif de transfert du produit élué (50).

7. Trousse selon la revendication 6, caractérisée en ce que la zone de réception du produit élué (15) présente plusieurs dispositifs de transfert du produit élué (50, 54, 55) qui sont chacun en contact fluidique avec la conduite de transport du liquide (11).

8. Trousse selon l'une des revendications précédentes, caractérisée en ce que la conduite de transport du liquide (11) se présente sous la forme d'une pièce intercalaire (16) faite d'un matériau stratifié capillaire et poreux, en ce que la pièce intercalaire 16 est inclue en sandwich entre une première pièce de recouvrement (17) et une seconde pièce de recouvrement (18) et en ce que les pièces de recouvrement (17, 18) ont chacune, dans la zone d'amenée de l'agent d'éluation (12) et la zone d'application des échantillons, au moins un creux (19, 20) à travers lequel une partie de la conduite de transport de liquide (11) conçue contre la pièce intercalaire (16) est accessible.

9. Trousse selon la revendication 8, caractérisée en ce que la pièce intercalaire (16') faite dans un matériau poreux stratifié est fixée sur une pièce porteuse (40) faite dans un matériau stratifié hydrofuge, pièce porteuse qui est inclue, avec la pièce intercalaire, entre les pièces de recouvrement (17', 18').

10. Trousse selon l'une des revendications précédentes, caractérisée en ce que les agents d'analyse contiennent un système réactif pour la détermination d'une élastase.
